# EUROPEAN PATENT APPLICATION

(11) **EP 4 691 406 A1**
(43) Date of publication of application: **11.02.2026**
(21) Application number: 24779962.0
(22) Date of filing: 22.03.2024
(51) Int. Cl.: A61B 34/30, B25J 15/04

(54) **SURGICAL ASSISTANCE ROBOT**

(30) Priority: 31.03.2023 JP 2023059583
(71) Applicant: Sony Group Corporation, Tokyo 108-0075 (JP)
(72) Inventor: WAKANA, Kazuhito, Tokyo 108-0075 (JP)
(74) Representative: D Young & Co LLP
(86) International application number: PCT/JP2024/011287
(87) International publication number: WO 2024/203844

(57) **Abstract**

A surgery support robot according to the present disclosure includes a base placed on a placement surface, a base arm including a plurality of links attached to the base and connected to each other, a follower arm base attached to the base arm, a follower arm attached to the follower arm base and including a tool driver to and from which a tool is attachable and detachable, and a turret on which a plurality of holders capable of holding the tool is formed and attached to the base arm or the follower arm base.

## Description

### Field

The present disclosure relates to a surgery support robot.

### Background

For example, Patent Literature 1 discloses a device that causes a robot itself to replace a tool attached to a distal end of an arm of the robot with another tool.

### Citation List

### Patent Literature

Patent Literature 1: JP 10-151592 A

### Summary

### Technical Problem

A tool for replacement is arranged at a position away from an arm and a base supporting the arm. When the tool is replaced, it is necessary to move a distal end of the arm to a position of the tool provided for replacement, so that a movement range of the arm and the tool is made large.

Here, as an example of a robot, there is a surgery support robot that performs surgery with a remotely manipulated arm. In the surgery support robot, when the movement range of the arm and the tool is made large, it takes time to replace the tool and surgery time increases. From the viewpoint of physical burden on a patient, it is preferable that the surgery time be short. When the movement range of the arm and the tool is made large, a risk that the arm and the tool interfere with the patient increases. From the viewpoint of safety of the patient, it is preferable that the movement range of the arm and the tool be small.

Therefore, the present disclosure proposes a surgery support robot capable of automatically replacing a tool while reducing time and improving safety. (In the following description, tool replacement performed by a robot itself without manual operation is referred to as automatic replacement.)

### Solution to Problem

A surgery support robot according to the present disclosure includes a base placed on a placement surface, a base arm including a plurality of links attached to the base and connected to each other, a follower arm base attached to the base arm, a follower arm attached to the follower arm base and including a tool driver to and from which a tool is attachable and detachable, and a turret on which a plurality of holders capable of holding the tool is formed and attached to the base arm or the follower arm base.

### Brief Description of Drawings

FIG. 1 is a diagram illustrating a schematic configuration of a surgery support robot according to a first embodiment of the present disclosure.
FIG. 2 is a perspective view of an operation device according to the first embodiment.
FIG. 3 is a partially enlarged perspective view in which a follower arm portion of the operation device is enlarged.
FIG. 4 is a partially enlarged perspective view in which a turret portion is enlarged.
FIG. 5 is a partially enlarged perspective view illustrating a turret in an exploded state.
FIG. 6 is an exploded perspective view of a holding unit.
FIG. 7 is a perspective view of the holding unit as seen from below.
FIG. 8 is a perspective view of a tool.
FIG. 9 is a partially enlarged side view in which a tool portion held by the holding unit is enlarged.
FIG. 10 is a plane view of the follower arm and the turret, and is a diagram illustrating a state during operation.
FIG. 11 is a plane view of the follower arm and the turret, and is a diagram illustrating a state when automatically replacing the tool.
FIG. 12 is a perspective view of the follower arm and the turret, and is a diagram illustrating a state when automatically replacing the tool.
FIG. 13 is a plane view of a turret according to a first variation.
FIG. 14 is a perspective view of the turret according to the first variation.
FIG. 15 is a perspective view of surroundings of a turret of an operation device according to a second variation.
FIG. 16 is a plane view of surroundings of the turret of the operation device according to the second variation.
FIG. 17 is a perspective view of the turret according to the second variation, and is a diagram illustrating a state when automatically replacing a tool.
FIG. 18 is a plane view of the turret according to the second variation, and is a diagram illustrating a state when automatically replacing the tool.
FIG. 19 is a perspective view of surroundings of a turret of an operation device according to a third variation.
FIG. 20 is a plane view of surroundings of the turret of the operation device according to the third variation.
FIG. 21 is a perspective view of the turret according to the third variation, and is a diagram illustrating a state when automatically replacing a tool.
FIG. 22 is a plane view of the turret according to the third variation, and is a diagram illustrating a state when automatically replacing the tool.
FIG. 23 is a perspective view of an operation device according to a second embodiment.
FIG. 24 is a partially enlarged perspective view in which a follower arm portion of the operation device according to the second embodiment is enlarged. Description of Embodiments

Hereinafter, embodiments of the present disclosure are described in detail with reference to the drawings. Note that, a surgery support robot according to the present disclosure is not limited to this embodiment. In the present specification and the drawings, components having substantially the same functional configuration are denoted by the same reference numeral, and redundant description is omitted.

One or a plurality of embodiments described below can each be implemented independently. In contrast, at least a part of a plurality of embodiments described below may be appropriately implemented in combination with at least a part of other embodiments. The plurality of embodiments may include novel features different from each other. Therefore, the plurality of embodiments can contribute to solving different objects or problems, and can exhibit different effects.

The present disclosure is described according to the following order of items.
1. First Embodiment
1-1. Outline of Surgery Support Robot
1-1-1. Configuration of Operation Device
1-1-2. Configuration of Manipulation Device
1-2. Effect of First Embodiment
1-3. First Variation
1-4. Second Variation
1-5. Third Variation
2. Second Embodiment
3. Appendix

### <1. First Embodiment>

### <1-1. Outline of Surgery Support Robot>

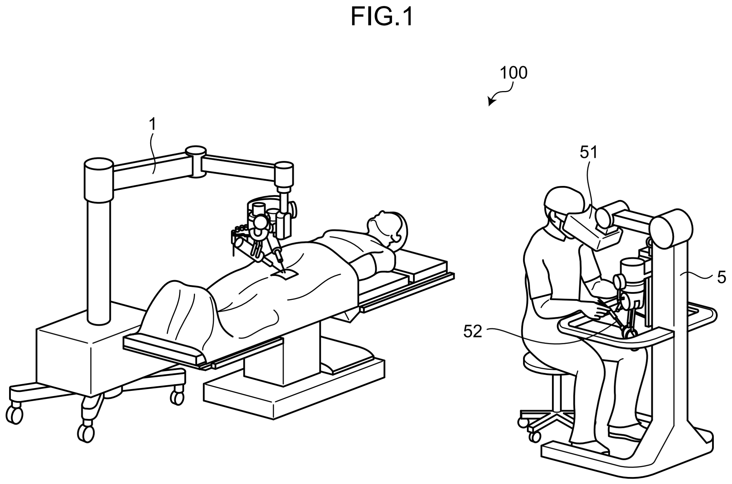

FIG. 1 is a diagram illustrating a schematic configuration of a surgery support robot according to a first embodiment of the present disclosure. A surgery support robot 100 includes an operation device 1 and a manipulation device 5. The operation device 1 is arranged in the vicinity of a patient who undergoes surgery. The operation device 1 includes an arm to which a tool necessary for surgery is attached. The manipulation device 5 is arranged at a place away from the operation device 1. The arm and tool provided in the operation device 1 can be operated by manipulating the manipulation device 5. A practitioner who performs surgery manipulates the manipulation device 5 to cause the operation device 1 to perform surgery from a place away from the patient.

### <1-1-1. Configuration of Operation Device>

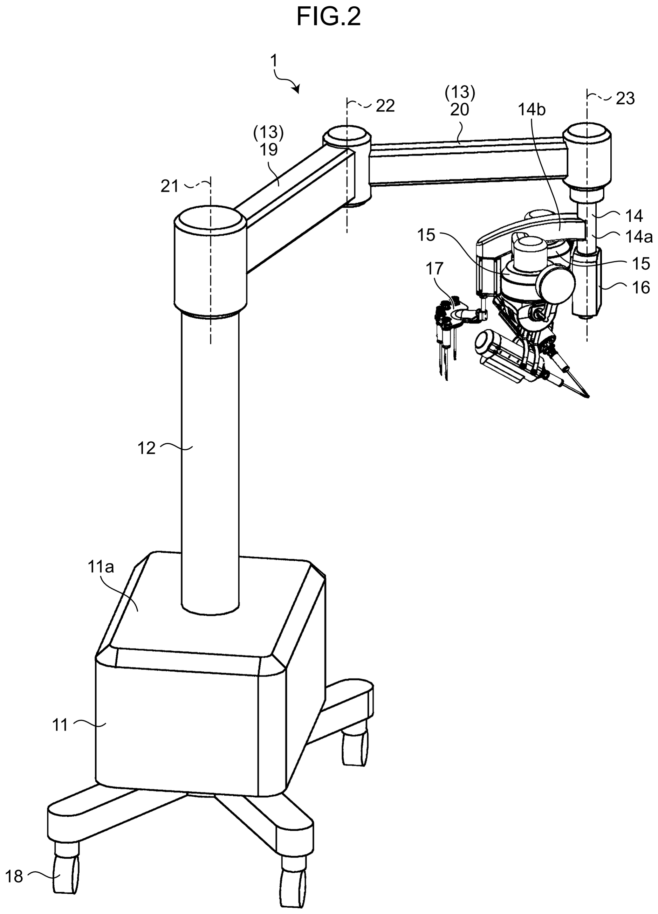

FIG. 2 is a perspective view of the operation device according to the first embodiment. The operation device 1 includes a base 11, a support 12, an arm 13, a follower arm base 14, a follower arm 15, a camera 16, and a turret 17.

The base 11 is a portion placed on a floor surface, which is a placement surface. A wheel 18 is attached to the base 11. Since the wheel 18 is attached, the operation device 1 can be easily moved. The support 12 is attached to a top surface 11a of the base 11. The support 12 is a columnar member extending vertically.

The arm 13 includes a first link 19 and a second link 20. The first link 19 is a rod-shaped member, and one end thereof is connected to the support 12. The first link 19 is connected to the support 12 so as to be rotatable about a rotation axis 21. The rotation axis 21 is an axis extending vertically. The first link 19 can rotate to move horizontally about the rotation axis 21. The first link 19 is attached at a position away from the top surface 11a of the base 11. The rotation of the first link 19 about the rotation axis 21 may be automatically performed by the manipulation of the manipulation device 5 or may be manually performed.

The second link 20 is a rod-shaped member, and one end thereof is connected to the other end of the first link 19. The second link 20 is connected to the first link 19 so as to be rotatable about a rotation axis 22. The rotation axis 22 is an axis extending vertically. The second link 20 can rotate to move horizontally about the rotation axis 22. The rotation of the second link 20 about the rotation axis 22 may be automatically performed by the manipulation of the manipulation device 5 or may be manually performed. Note that, the number of links included in the arm 13 is not limited two and may be one or three or more. As long as the follower arm 15 to be described later can be moved close to a surgical site of the patient with the base 11 kept fixed, the rotation axis of the link is not limited to a vertically extending example. Note that, the links 20 and 21 may be connected so as to be linearly movable.

The follower arm base 14 is connected to the other end of the second link 20. The follower arm base 14 is connected to the second link 20 so as to be rotatable about a rotation axis 23. The follower arm base 14 includes a first portion 14a extending downward along the rotation axis 23 and a second portion 14b branched from the first portion 14a. When the first portion 14a rotates about the rotation axis 23, the second portion 14b can rotate to move horizontally. The rotation of the follower arm base 14 about the rotation axis 23 may be automatically performed by the manipulation of the manipulation device 5 or may be manually performed. Note that, the follower arm base 14 may be fixed to the second link 20.

The operation device 1 includes two follower arms 15. The follower arm 15 is fixed to and supported by the second portion 14b of the follower arm base 14.

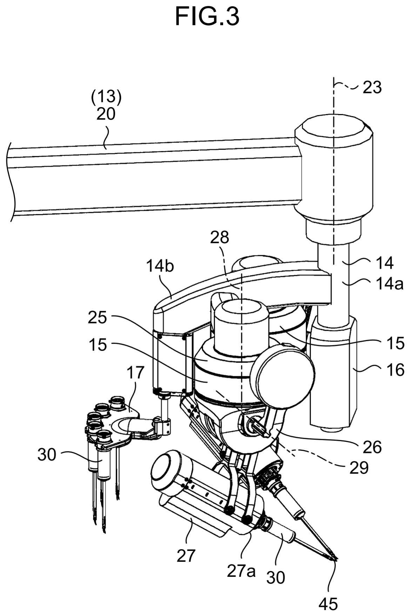

FIG. 3 is a partially enlarged perspective view in which a follower arm portion of the operation device is enlarged. Each of the follower arms 15 includes a root side driver 25, a distal end side driver 26, and a tool driver 27. The root side driver 25 is fixed to the side of the follower arm base 14. The distal end side driver 26 is attached below the root side driver 25.

The root side driver 25 rotates the distal end side driver 26 about a drive axis 28. The drive axis 28 is an axis extending vertically, and is a second drive axis. The distal end side driver 26 rotates horizontally about the drive axis 28. The rotation of the distal end side driver 26 about the drive axis 28 is automatically performed by the manipulation of the manipulation device 5. For example, an electric motor is provided on the root side driver 25, and the electric motor is driven on the basis of the manipulation of the manipulation device 5, and the distal end side driver 26 rotates.

The tool driver 27 is attached below the distal end side driver 26. The distal end side driver 26 rotates the tool driver 27 about a drive axis 29. The drive axis 29 is an axis extending horizontally, and is a first drive axis. The tool driver 27 vertically rotates about the drive axis 29. The rotation of the tool driver 27 about the drive axis 29 is automatically performed by the manipulation of the manipulation device 5. For example, an electric motor is provided on the distal end side driver 26, and the electric motor is driven on the basis of the manipulation of the manipulation device 5, and the tool driver 27 rotates. When the root side driver 25 rotates the distal end side driver 26 about the drive axis 28, the tool driver 27 also rotates horizontally. The follower arm 15 is a unit including the drive axes 28 and 29, which are two drive axes from the tool driver 27 side, among a plurality of axes for driving the tool driver 27.

A tool 30 is detachably attached to an attachment portion 27a provided at a distal end of the tool driver 27. An end effector 45 is provided at a distal end of the tool 30. In surgery, the end effector 45 is required to perform various actions. Examples of the required actions include, for example, an action of sandwiching to hold an affected site, an action of resecting the affected site, and an action of holding a surgical tool such as a needle or a thread. That is, the end effector 45 is required to perform an action in place of various surgical tools such as tweezers, scissors, and a needle holder. A plurality of types of tools 30 including different end effectors 45 is prepared according to the required actions, and various actions can be performed by replacing the tools 30 attached to the tool driver 27. The tool driver 27 is rotated about the drive axes 28 and 29 to move a position of the tool 30. The tool driver 27 includes an actuator (not illustrated) that operates the end effector 45. The end effector 45 preferably has one-degree-of-freedom for gripping in addition to two-degree-of-freedom for wrist joint, but it is also possible to have no degree-of-freedom depending on the required action.

In this first embodiment, two follower arms 15 are provided. The two follower arms 15 are provided on both sides sandwiching the second portion 14b of the follower arm base 14. In the operation device 1, surgery can be performed using the two tool drivers 27. The number of follower arms 15 may be one.

The camera 16 is provided at a lower end of the first portion 14a of the follower arm base 14. The camera 16 captures an image below the same and transmits the captured image to the manipulation device 5. A range captured by the camera 16 is an operational field. During the operation, the tool driver 27 is moved so that the tool 30 enters the operational field below the camera 16. Since the follower arm 15 and the camera 16 are fixed to the same follower arm base 14, a positional relationship between the operational field and the follower arm 15 does not change.

That is, it can be said that the follower arm 15 is a unit that can drive the drive axis 29, which is the first drive shaft, and the drive axis 28, which is the second drive shaft, to move only the tool driver 27 without changing the range in the operational field.

The turret 17 is attached to the second portion 14b of the follower arm base 14 via a moving mechanism 31. The turret 17 is attached to a distal end of the second portion 14b, and is attached to an opposite side of the camera 16 across the follower arm 15. That is, the turret 17 and the moving mechanism 31 are provided on an opposite side of the operational field with respect to the follower arm 15. Note that, the turret 17 may be directly attached to the second portion 14b.

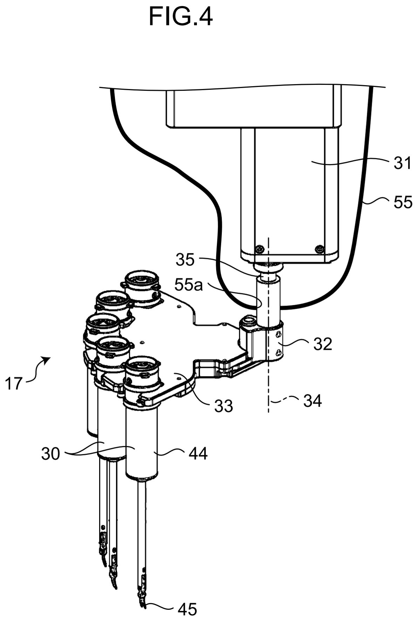

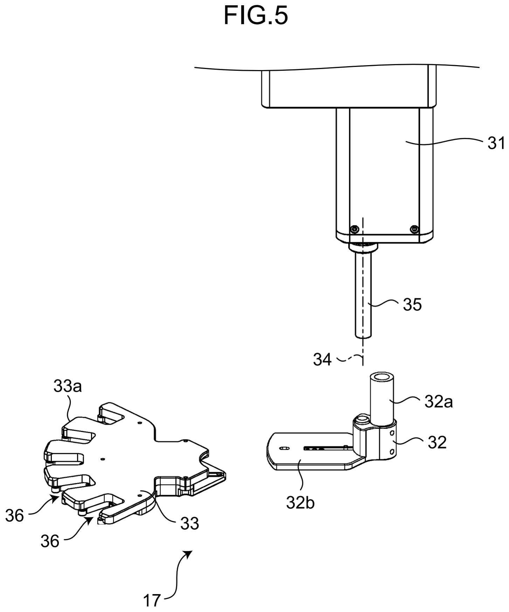

FIG. 4 is a partially enlarged perspective view in which a turret portion is enlarged. FIG. 5 is a partially enlarged perspective view illustrating the turret in an exploded state. The moving mechanism 31 is fixed to the second portion 14b of the follower arm base 14. The moving mechanism 31 includes an output shaft 35 that rotates about a vertically extending drive axis 34. For example, an electric motor not illustrated is provided in the moving mechanism 31, and the output shaft 35 is rotated by the electric motor.

The turret 17 includes a detachable portion 32 and a holding unit 33. A first portion 32a into which the output shaft 35 of the moving mechanism 31 is inserted is formed in the detachable portion 32. A hole into which the output shaft 35 is inserted is formed in the first portion 32a. The output shaft 35 is inserted into the hole of the first portion 32a, so that the detachable portion 32 is attached to the moving mechanism 31. The detachable portion 32 rotates together with the output shaft 35. A second portion 32b to which the holding unit 33 is attached is formed in the detachable portion 32.

The holding unit 33 holds a plurality of tools 30 for replacement. The holding unit 33 is a plate-shaped member. The holding unit 33 has a fan-shaped plate shape including an arc-shaped outer edge 33a in plane view. The arc-shaped outer edge 33a overlaps a part of a circle centered on the drive axis 34. A plurality of holders 36 that holds the tools 30 is formed side by side on the outer edge 33a of the holding unit 33. In other words, the holder 36 is formed on an arc centered on the drive axis 34. Since a plurality of holders 36 is formed, the holding unit 33 can hold the plurality of tools 30 for replacement.

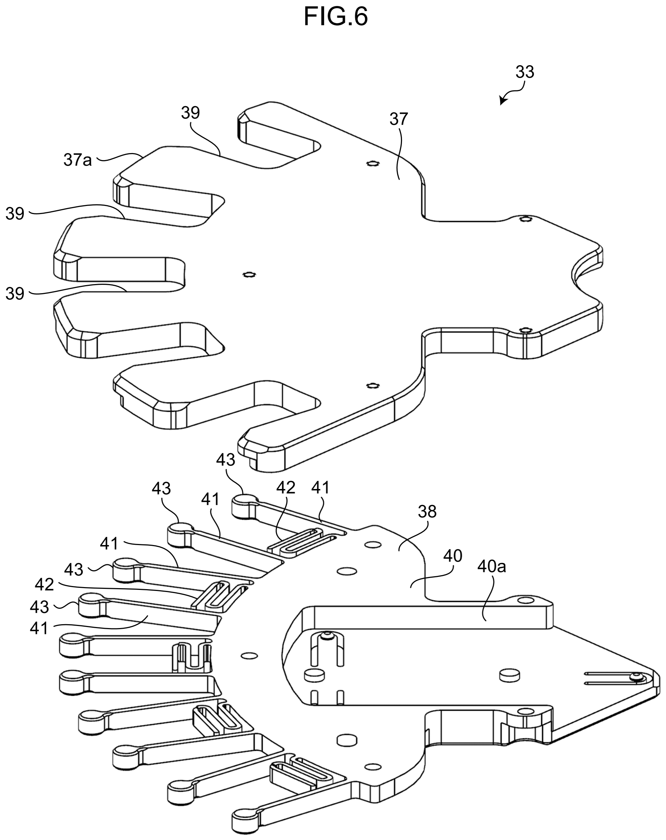

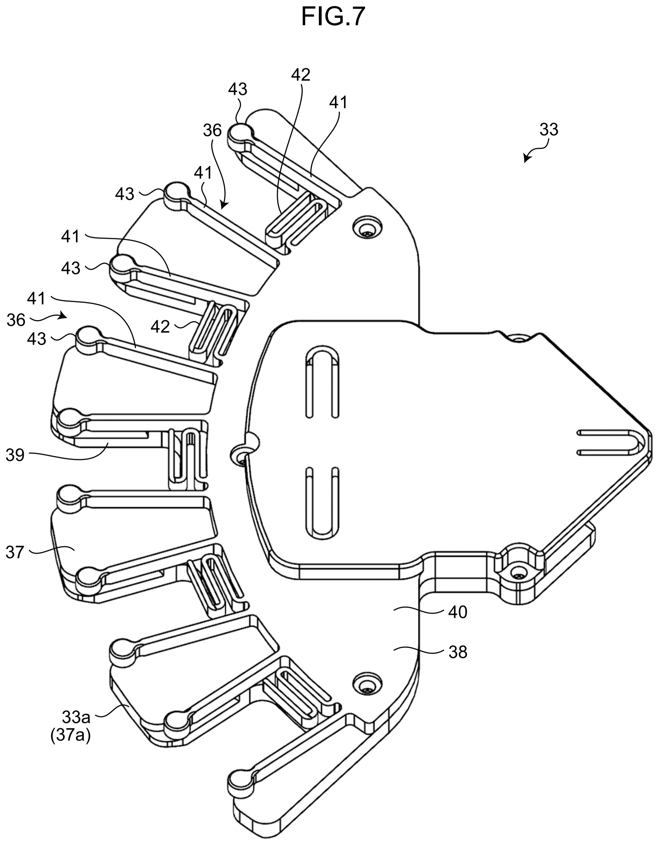

FIG. 6 is an exploded perspective view of the holding unit. FIG. 7 is a perspective view of the holding unit as seen from below. The holding unit 33 includes a first plate portion 37 and a second plate portion 38. The first plate portion 37 and the second plate portion 38 overlap each other in a thickness direction of the holding unit 33 having a plate shape. The outer edge 33a of the holding unit 33 described above serves as an outer edge 37a of the first plate portion 37.

The first plate portion 37 has a fan shape. A groove 39 is formed at a position corresponding to the holder 36 on the outer edge 37a of the first plate portion 37. That is, a plurality of grooves 39 is formed side by side on the outer edge 37a of the first plate portion 37. The groove 39 is formed to have a width in which the tool 30 can be inserted. The width of the groove 39 is described in detail later.

The second plate portion 38 includes a base portion 40, a leaf spring 41, and an elastic portion 42. The base portion 40 is a portion attached to the second portion 32b of the detachable portion 32. The base portion 40 is provided in a region not overlapping the groove 39 formed in the first plate portion 37 in plane view.

A groove 40a is formed in the base portion 40. By overlapping the first plate portion 37 and the second plate portion 38, an upper portion of the groove 40a is closed by the first plate portion 37, and a hole into which the second portion 32b of the detachable portion 32 is inserted is formed. That is, the second portion 32b is inserted into the groove 40a formed in the second plate portion 38, so that the holding unit 33 is attached to the detachable portion 32. Note that, the hole into which the second portion 32b is inserted may be formed of a groove formed in the first plate portion 37, or may be formed of a groove formed in the first plate portion 37 and the second plate portion 38.

The leaf spring 41 is formed so as to extend from the base portion 40 toward a radially outer side of the fan shape. A plurality of leaf springs 41 is formed so as to be located on both sides of the groove 39 formed in the first plate portion 37 in a state in which the first plate portion 37 and the second plate portion 38 overlap each other. When the leaf spring 41 is deformed so as to expand outward in a width direction of the groove 39, this exerts an elastic force in a direction returning inward in the width direction by a restoring force. A widened portion 43 having a widened width is formed at a distal end of the leaf spring 41. An interval between the adjacent leaf springs 41 and an interval between the adjacent widened portions 43 are described in detail later. Note that, the holding unit 33 and the detachable portion 32 may be integrally formed.

The elastic portion 42 is provided between the roots of the leaf springs 41, and when compressed in a direction of the base portion 40, this exerts an elastic force in a direction away from the base portion 40 by a restoring force. When the detachable portion 32 is rotated by the moving mechanism 31, the holding unit 33 rotates horizontally.

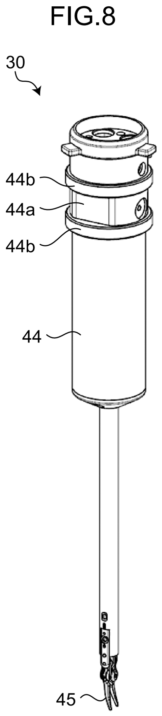

FIG. 8 is a perspective view of the tool. The tool 30 includes a root 44 and the end effector 45. The root 44 is a portion to be attached to and detached from the tool driver 27. The root 44 has a tubular shape. The root 44 is a portion held by the holding unit 33. Parallel surfaces 44a parallel to each other are formed in the root 44, and protrusions 44b protruding from a cylindrical surface are formed on both sides across the parallel surfaces 44a.

The end effector 45 is a portion that is provided at one end of the root 44 and performs an action necessary for surgery, such as sandwiching and holding an affected site, resecting the affected site, or holding a surgical tool such as a needle or a thread, as described above.

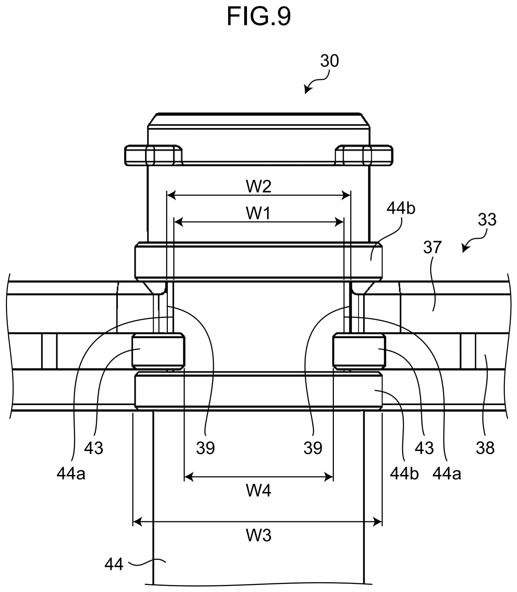

FIG. 9 is a partially enlarged side view in which a tool portion held by the holding unit is enlarged. In FIG. 9, the tool 30 is inserted into the holder 36. A width W1 between the parallel surfaces 44a formed in the tool 30 is narrower than a width W2 of the groove 39 formed in the first plate portion 37. Therefore, the portion of the tool 30 in which the parallel surfaces 44a are formed can be inserted into the holder 36.

An outer diameter W3 of the protrusion 44b formed in the tool 30 is larger than the width W2 of the groove 39. Therefore, vertical movement of the tool 30 inserted into the holder 36, with the protrusion 44b caught by the first plate portion 37, is restricted. This prevents removal of the tool 30 from the holder 36.

A distance W4 between the widened portions 43 formed at the distal ends of the adjacent leaf springs 41 is narrower than the width W1 between the parallel surfaces 44a of the tool 30. By allowing the tool 30 to abut a space between the widened portions 43 and then further pushing the same, the widened portion 43 can be expanded and the tool 30 can be inserted into the holder 36. When the tool 30 passes between the widened portions 43 and is inserted into the holder 36, the leaf spring 41 is restored to its original shape. Since the distance W4 between the widened portions 43 is narrower than the width W1 between the parallel surfaces 44a, removal of the tool 30 in a front direction of a paper surface of FIG. 9 is prevented.

Referring back to FIGS. 6 and 7, the tool 30 inserted into the holder 36 abuts the elastic portion 42 to push the elastic portion 42. A biasing force for moving the tool 30 to a front side of the paper surface of FIG. 9 is applied by the restoring force of the elastic portion 42. The tool 30 is sandwiched between the elastic portion 42 and the widened portion 43 by a biasing force from the elastic portion 42. As a result, the tool 30 is positioned in a depth direction of the paper surface of FIG. 9. The leaf spring 41 and the elastic portion 42 are positioning units for positioning the tool 30 inserted into the groove 39. A function of the holder 36 that holds the tool 30 is implemented by cooperation of the groove 39, the leaf spring 41, and the elastic portion 42.

Note that, the positioning of the tool 30 inserted into the holder 36 is not limited to a configuration performed by the leaf spring 41 and the elastic portion 42. For example, a magnet may be provided in the holder 36 instead of the leaf spring 41 and the elastic portion 42. Positioning can be performed by attraction of the tool 30 inserted into the holder 36 to the magnet. In this case, the root 44 of the tool 30 may be formed of metal to be attracted, or a magnet may also be provided to the root 44 of the tool 30 to be attracted.

### <Operation When Automatically Replacing Tool>

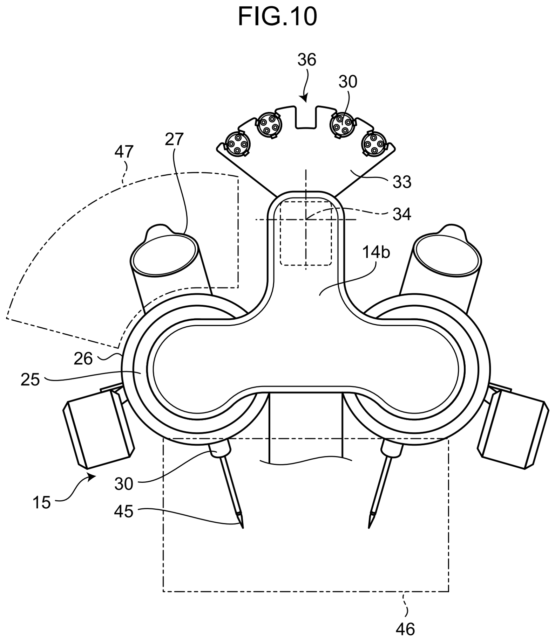

FIG. 10 is a plane view of the follower arm and the turret, and is a diagram illustrating a state during the operation. The tool driver 27 during the operation takes a posture in which the end effector 45 enters the operational field 46. During the operation, the holding unit 33 of the turret 17 is arranged at a position avoiding a range where the tool driver 27 moves. Therefore, the movement of the tool driver 27 during the operation is not inhibited by the holding unit 33. In FIG. 10, an approximate range in which the tool driver 27 moves during the operation is illustrated as a movement range 47.

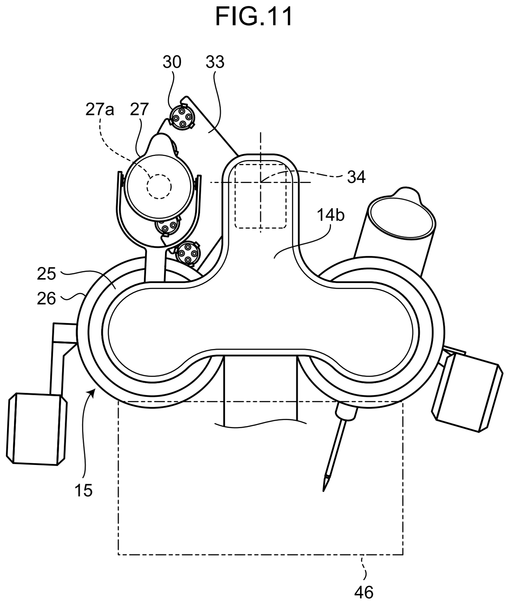

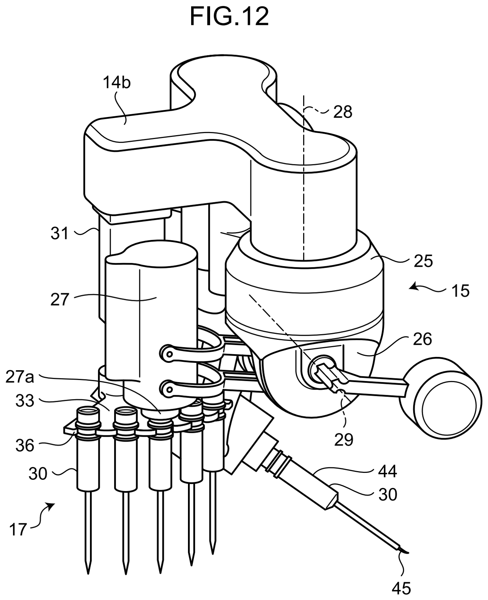

FIG. 11 is a plane view of the follower arm and the turret, and is a diagram illustrating a state when automatically replacing the tool. FIG. 12 is a perspective view of the follower arm and the turret, and is a diagram illustrating a state when automatically replacing the tool.

When the tool 30 attached to the tool driver 27 is replaced with the tool 30 held by the turret 17, as is the case with the tool driver 27 on a left side of a paper surface of FIG. 11, this is moved to an opposite side of the operational field 46 across the root side driver 25 and the distal end side driver 26. The holding unit 33 of the turret 17 is rotated about the drive axis 34, and the holder 36 in which the tool 30 is not held is moved to a position overlapping the attachment portion 27a of the tool driver 27. At that time, as illustrated in FIG. 12, the root 44 of the tool driver 27 is moved to a position not interfering with the holding unit 33.

The tool driver 27 causes the holder 36 to hold the tool 30 attached to the attachment portion 27a and removes the tool 30. Next, the holding unit 33 is rotated, and the holder 36 holding the tool 30 to be used next is moved to the position overlapping the attachment portion 27a. The tool driver 27 attaches the tool 30 held by the holder 36 to the attachment portion 27a and returns to an operation position. At that time, the holding unit 33 is also rotated to move to a position outside the movement range 47. That is, a state returns to the state illustrated in FIG. 10.

### <1-1-2. Configuration of Manipulation Device>

Referring back to FIG. 1, the manipulation device 5 includes an image display device 51 and a manipulation unit 52. An image captured by the camera 16 of the operation device 1 is displayed on the image display device 51. The tool driver 27 and the end effector 45 of the operation device 1 can be driven by manipulating the manipulation unit 52. The practitioner manipulates the manipulation unit 52 while checking the image displayed on the image display device 51, and performs surgery on a patient at a distant place by the operation device 1. The above-described automatic replacement of the tool 30 is performed by instructing the operation device 1 to replace the tool 30 attached to the tool driver 27 through the manipulation device 5.

### <1-2. Effect of First Embodiment>

In the surgery support robot 10 according to the first embodiment described above, the follower arm 15 and the turret 17 are attached to the same follower arm base 14. As a result, a distance between the follower arm 15 and the turret 17 decreases. As the distance between the follower arm 15 and the turret 17 decreases, the tool 30 can be automatically replaced only by moving the tool driver 27 without moving the follower arm base 14 on which the follower arm 15 is supported. As a result, the movement range of the tool driver 27 at the time of automatic replacement of the tool 30 can be made small. Since the movement range of the tool driver 27 is made small, a time required for automatic replacement of the tool 30 can be reduced. The reduction in time required for automatic replacement of the tool 30 leads to a reduction in surgery time. Therefore, it is possible to reduce a physical burden on a patient by reducing the surgery time. Since the movement range of the tool driver 27 is made small, a risk that the tool 30 interferes with the patient is reduced. Therefore, safety of the patient can be improved.

Also when the tool 30 is automatically replaced, a relative positional relationship between the follower arm base 14 supporting the follower arm 15 and the operational field does not change. In a case where the two follower arms 15 are provided as in the first embodiment, even while the tool 30 is replaced by the tool driver 27 provided in one follower arm 15, the surgery can be continued by the tool driver 27 provided in the other follower arm 15. Therefore, it is possible to reduce the time during which the surgery is interrupted for replacing the tool 30, that is, reduce the surgery time, and reduce the physical burden on the patient.

In a case where a member that holds the tool 30 for replacement is placed at a position away from the base, for example, on the floor, a positional relationship between the member that holds the tool 30 for replacement and the follower arm 15 also changes each time the operation device 1 is moved. Therefore, each time the operation device 1 is moved, it is necessary to perform calibration work of teaching the operation device 1 the position of the member that holds the tool 30 for replacement. In contrast, in the operation device 1 of the first embodiment, since a relative positional relationship between the turret 17 that holds the tool 30 for replacement and the follower arm 15 does not change, the above-described calibration work is not necessary.

By rotating the fan-shaped holding unit 33, the holder 36 can be moved to the movement range of the two tool drivers 27. As a result, it is possible to cause the two tool drivers 27 to automatically replace the tool 30 with one holding unit, so that it is possible to reduce a size of the device and a manufacturing cost as compared with a case where the holding unit 33 is provided for each tool driver 27.

Since the holding unit 33 is attachable to and detachable from the output shaft 35 of the moving mechanism 31, it is easy to change a plurality of holding units 33 holding different tools 30 according to a type and a process of surgery.

Since a part of the turret 17, specifically, the detachable portion 32 and the holding unit 33 are attachable and detachable, it is possible to detach the detachable portion 32 and the holding unit 33 from the operation device 1 to perform sterilization treatment. For example, when the detachable portion 32 and the holding unit 33 are formed of polypropylene, polymethylpentene, or polytetrafluoroethylene, sterilization treatment by an autoclave can be performed. For example, when the detachable portion 32 and the holding unit 33 are formed of polytetrafluoroethylene, polysulfone, or polymethylpentene, dry sterilization treatment can be performed.

Since the detachable portion 32 and the holding unit 33 are attachable and detachable, the detachable portion 32 and the holding unit 33 that have been used can be discarded and replaced with new ones to ensure cleanliness.

In the holding unit 33, a structure for holding the tool 30 is a mechanical structure such as the leaf spring 41, and no electrical component is used. Therefore, the structure is simple and the sterilization treatment can be easily performed. The manufacturing cost is easily reduced, and it is easy to adopt an operation of discarding the used holding unit 33.

As illustrated in FIG. 4, in a case where the operation device 1 is covered with a drape 55 in order to secure a clean area, the holding unit 33 that holds the tool 30 for replacement needs to be exposed. In such a case, if the detachable portion 32 and the holding unit 33 are attachable and detachable, when the drape 55 covers in a state in which the detachable portion 32 and the holding unit 33 are detached, the drape 55 is only required to have an opening 55a having a size that allows the output shaft 35 to pass therethrough. In a case where the detachable portion 32 and the holding unit 33 cannot be detached, it is necessary to perform work of forming an opening having a size allowing the holding unit 33 to pass therethrough, covering the same with the drape 55, and then closing a gap between the opening 55a and the output shaft 35. In the operation device 1 of the first embodiment, such work is not necessary. The detachable portion 32 and the holding unit 33 can be replaced without detaching the drape 55.

### <1-3. First Variation>

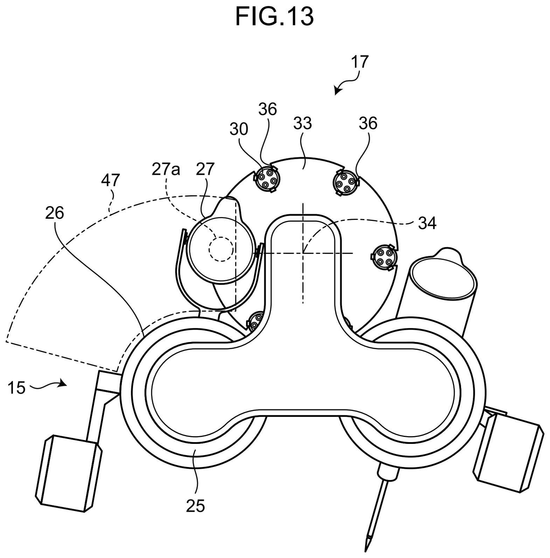

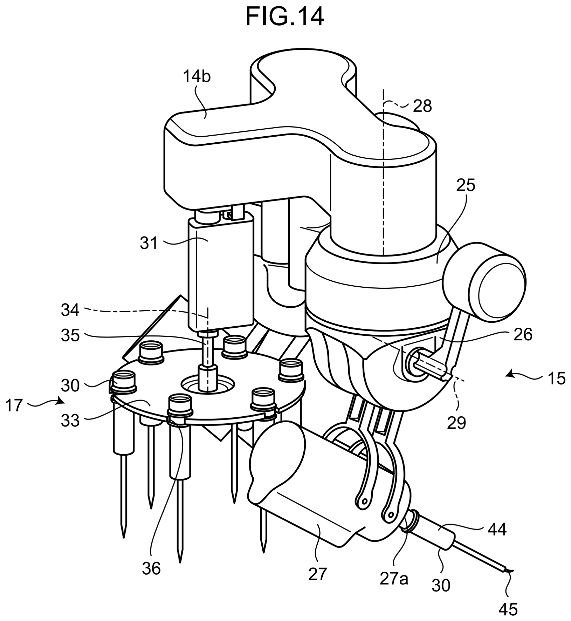

FIG. 13 is a plane view of a turret according to a first variation. FIG. 14 is a perspective view of the turret according to the first variation. In a turret 17 according to the first variation, a holding unit 33 has a circular shape centered on a drive axis 34. A plurality of holders 36 is formed side by side on an outer edge 33a of the circular holding unit 33. Although a detailed configuration is omitted, also in the first variation, the holding unit 33 is configured such that a first plate portion and a second plate portion overlap each other.

By rotating the holding unit 33 about the drive axis 34, a tool 30 to be used next can be moved to a position overlapping an attachment portion 27a when the tool 30 is automatically replaced. Note that, in a case where the holding unit 33 has the circular shape, a region occupied by the holding unit 33 does not change even when the holding unit 33 is rotated. Therefore, a part of the holding unit 33 overlaps a movement range 47 of the tool driver 27 during operation. Therefore, in a case where the holding unit 33 is formed in the circular shape as in variation 1, a range in which the tool driver 27 can be moved is smaller as compared with a case where the holding unit 33 is formed in an arc shape. In contrast, in a case where the holding unit 33 is formed in the circular shape as in variation 1, the outer edge 33a is longer than that of the arc-shaped holding unit 33, so that the number of holders 36 that can be formed increases. Therefore, by making the holding unit 33 circular as in variation 1, the number of tools 30 that can be held by the holding unit 33 can be increased.

### <1-4. Second Variation>

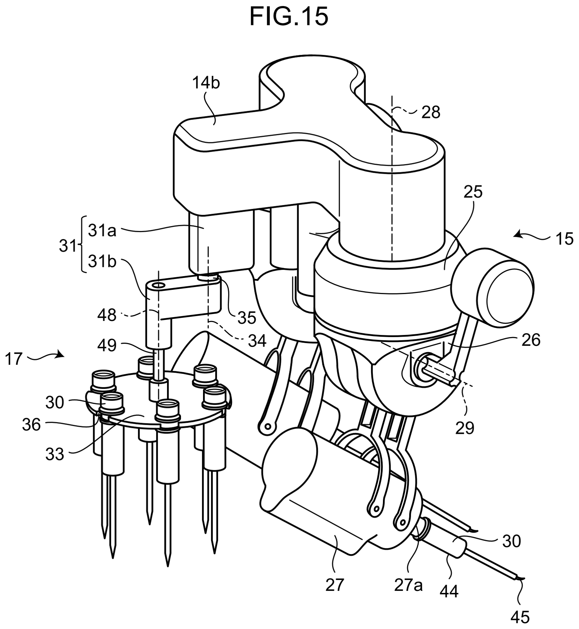

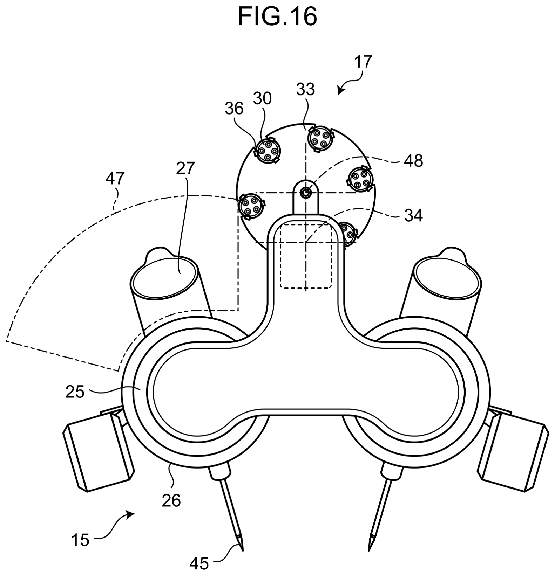

FIG. 15 is a perspective view of surroundings of a turret of an operation device according to a second variation. FIG. 16 is a plane view of surroundings of the turret of the operation device according to the second variation. In the second variation, a moving mechanism 31 includes a first moving mechanism 31a and a second moving mechanism 31b. A drive axis 34 and an output shaft 35 are provided in the first moving mechanism 31a.

The second moving mechanism 31b is attached to the output shaft 35 of the first moving mechanism 31a. The second moving mechanism 31b includes an output shaft 49 that rotates about a vertically extending drive axis 48. A holding unit 33 is attached to the output shaft 49. Both the drive axis 34 and the drive axis 48 are vertically extending axes, but are eccentric to each other.

The holding unit 33 has a circular shape as in the first variation, and is formed by overlapping a first plate portion and a second plate portion. The center portion is attached to the output shaft 49. When the output shaft 49 rotates, the holding unit 33 rotates about the drive axis 48, but a relative positional relationship between the holding unit 33 and a follower arm 15 does not change. In contrast, when the output shaft 35 rotates, the holding unit 33 rotates to move about the drive axis 34. At that time, a relative positional relationship between the holding unit 33 and the follower arm 15 changes.

As illustrated in FIGS. 15 and 16, by moving the holding unit 33 to a position away from the follower arm 15 during operation, the holding unit 33 can be arranged at a position outside a movement range 47 of the tool driver 27. In this state, the range in which the tool driver 27 can move during the operation can be made large, but the tool 30 cannot be automatically replaced.

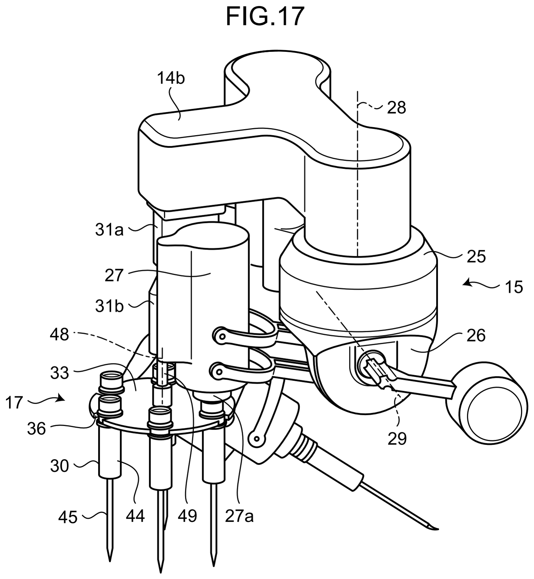

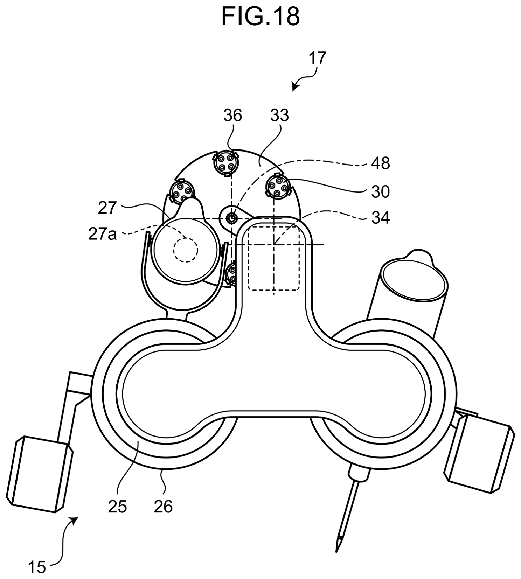

FIG. 17 is a perspective view of the turret according to the second variation, and is a diagram illustrating a state when automatically replacing the tool. FIG. 18 is a plane view of the turret according to the second variation, and is a diagram illustrating a state when automatically replacing the tool.

As illustrated in FIGS. 17 and 18, when the tool 30 is automatically replaced, the holding unit 33 is rotated to be moved about the drive axis 34 to bring the holding unit 33 closer to the follower arm 15. By bringing the holding unit 33 closer to the follower arm 15, the holder 36 can be moved to a position overlapping an attachment portion 27a of the tool driver 27, and the tool 30 can be automatically replaced.

In this manner, by providing the moving mechanism 31 with the two eccentric drive axes 34 and 48, it is possible to make the range in which the tool driver 27 can move during the operation large even in a case where the circular holding unit 33 is used. By forming the holding unit 33 in the circular shape, it is possible to hold more tools 30 as compared with a fan-shaped holding unit 33. Note that, the fan-shaped holding unit 33 may be combined with a configuration in which the moving mechanism 31 is provided with the two eccentric drive axes 34 and 48.

### <1-5. Third Variation>

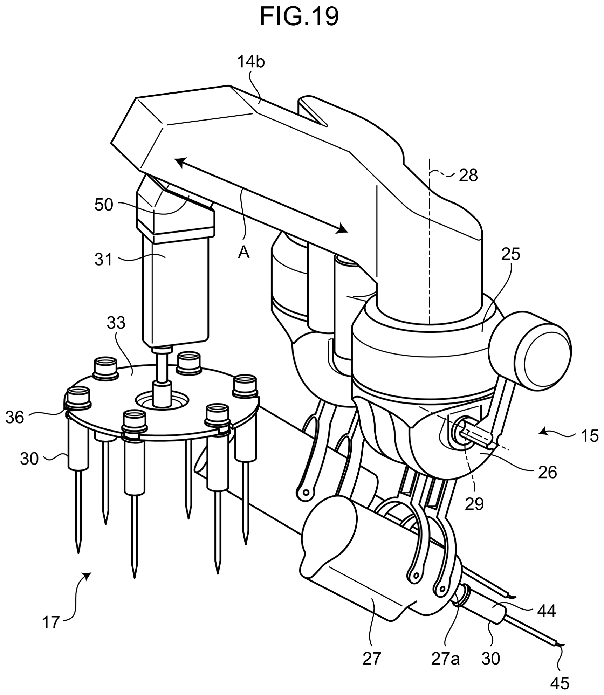

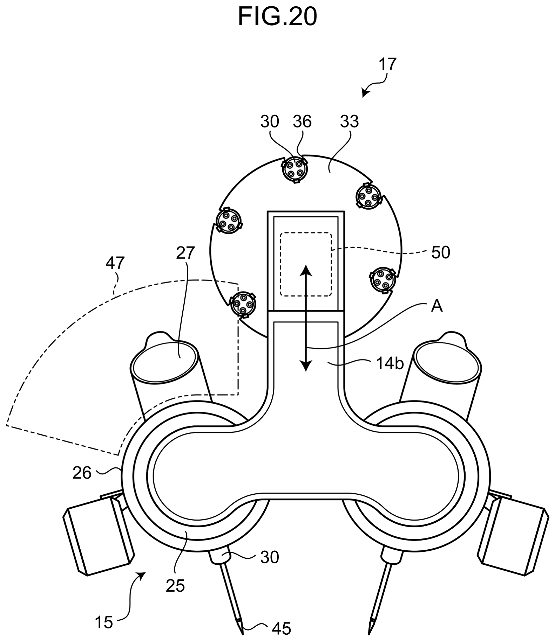

FIG. 19 is a perspective view of surroundings of a turret of an operation device according to a third variation. FIG. 20 is a plane view of surroundings of the turret of the operation device according to the third variation. In the third variation, in addition to a drive axis 34 and an output shaft 35, a moving mechanism 31 includes a linear motion unit 50 that linearly moves in a direction in which a second portion 14b of a follower arm base 14 extends. The linear motion unit 50 is driven by, for example, a linear motion type actuator or a linear motor that converts rotational motion of an electric motor into linear motion. The linear motion unit 50 allows a turret 17 to move in a direction away from a follower arm 15 and in a direction approaching the follower arm 15. The holding unit 33 has a circular shape as in the first variation, and is formed by overlapping a first plate portion and a second plate portion. The center portion is attached to an output shaft 35.

When the output shaft 35 rotates, the holding unit 33 rotates about the drive axis 34, but a relative positional relationship between the holding unit 33 and the follower arm 15 does not change. In contrast, when the linear motion unit 50 moves, a relative positional relationship between the holding unit 33 and the follower arm 15 changes.

As illustrated in FIGS. 19 and 20, by moving the holding unit 33 to a position away from the follower arm 15 during operation, the holding unit 33 can be arranged at a position outside a movement range 47 of a tool driver 27. In this state, the range in which the tool driver 27 can move during the operation can be made large, but the tool 30 cannot be automatically replaced.

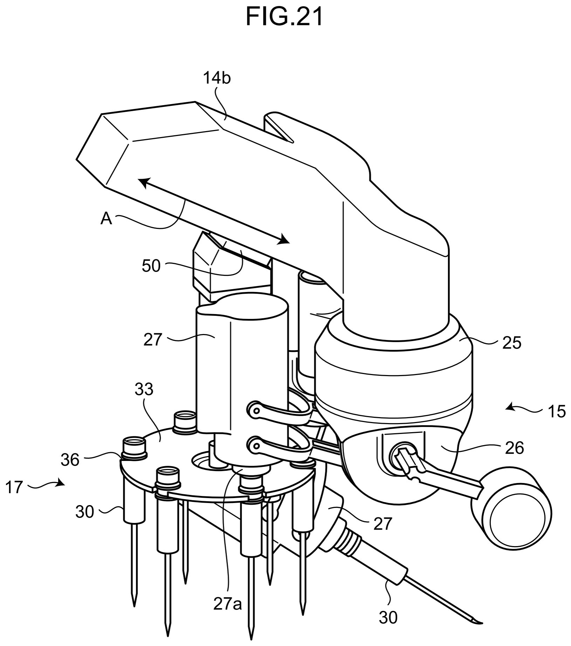

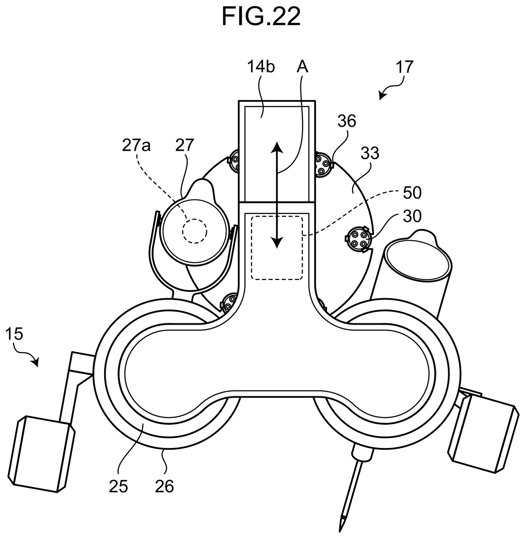

FIG. 21 is a perspective view of the turret according to the third variation, and is a diagram illustrating a state when automatically replacing the tool. FIG. 22 is a plane view of the turret according to the third variation, and is a diagram illustrating a state when automatically replacing the tool.

As illustrated in FIGS. 21 and 22, when the tool 30 is automatically replaced, the linear motion unit 50 is moved to bring the holding unit 33 closer to the follower arm 15. By bringing the holding unit 33 closer to the follower arm 15, the holder 36 can be moved to a position overlapping an attachment portion 27a of the tool driver 27, and the tool 30 can be automatically replaced.

In this manner, by providing the moving mechanism 31 with the linear motion unit 50 that linearly moves, it is possible to make the range in which the tool driver 27 can move during the operation large even in a case where the circular holding unit 33 is used. By forming the holding unit 33 in the circular shape, it is possible to hold more tools 30 as compared with a fan-shaped holding unit 33. Note that, the fan-shaped holding unit 33 may be combined with a configuration in which the moving mechanism 31 is provided with the linear motion unit 50.

### <2. Second Embodiment>

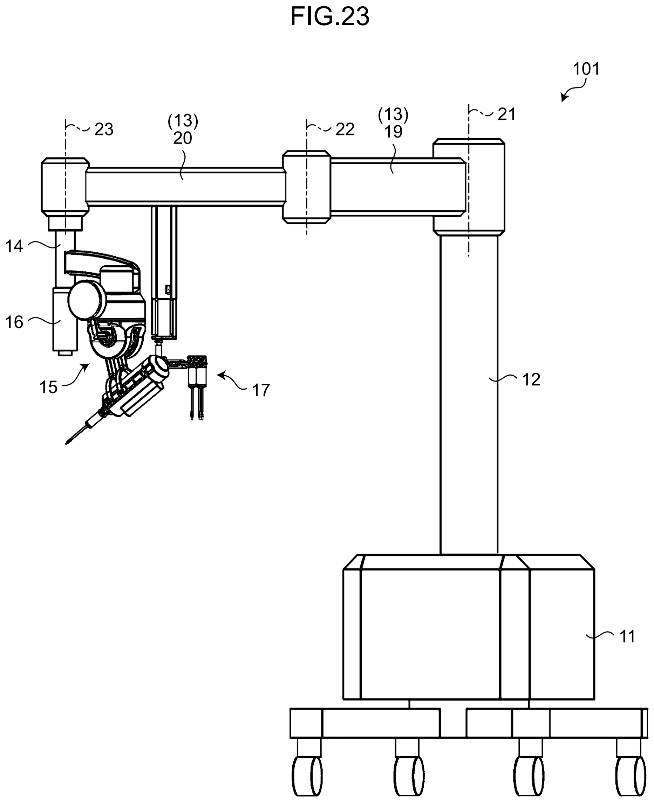

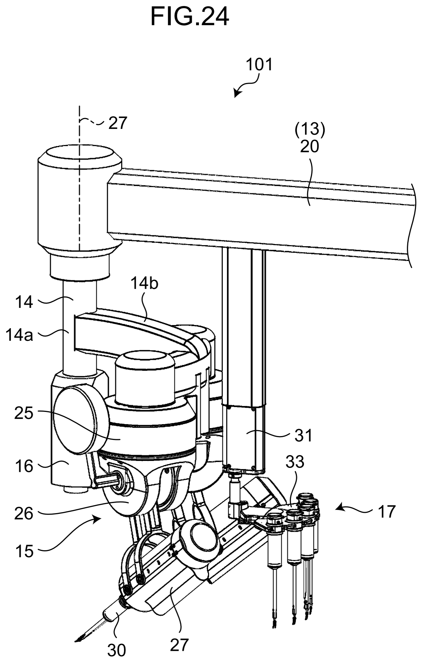

FIG. 23 is a perspective view of an operation device according to a second embodiment. FIG. 24 is a partially enlarged perspective view in which a follower arm portion of the operation device according to the second embodiment is enlarged. Note that, a configuration similar to that in the above-described first embodiment is denoted by the similar reference numeral, and a detailed description thereof is omitted.

In an operation device 101 according to the second embodiment, a moving mechanism 31 and a turret 17 are attached to a second link 20. As in the first embodiment, the moving mechanism 31 and the turret 17 are attached so as to be able to be arranged at positions opposite to a camera 16 across the follower arm 15. The second embodiment is different from the first embodiment in that relative positions of the follower arm 15 and the turret 17 change when the follower arm base 14 is rotated about a rotation axis 23. Therefore, although a posture of the follower arm base 14 for arranging the turret 17 near the follower arm 15 is limited, a movement range of a tool driver 27 when the tool 30 is automatically replaced is made smaller as compared with a case where the tool for replacement is arranged on a base 11 or the floor, and replacement time of the tool 30 is reduced, that is, operation time is reduced. Since the movement range of the tool driver 27 is made small, a risk of interfering with the patient is reduced, so that safety is improved. Note that, when the moving mechanism 31 and the turret 17 are fixed not only to a second link 20 but also to a base arm 13, an effect such as reducing the replacement time of the tool 30 can be obtained.

### <3. Appendix>

Note that, the present technology can also have the following configurations.
(1) A surgery support robot comprising:
   a base placed on a placement surface;
   a base arm including a plurality of links attached to the base and connected to each other;
   a follower arm base attached to the base arm;
   a follower arm attached to the follower arm base and including a tool driver to and from which a tool is attachable and detachable; and
   a turret on which a plurality of holders capable of holding the tool is formed and attached to the base arm or the follower arm base.
(2) The surgery support robot according to claim (1), comprising
   a plurality of follower arms, wherein
   each of the plurality of follower arms includes the tool driver.
(3) The surgery support robot according to (2), wherein
   the plurality of the tool drivers is capable of mounting the tool held by a common turret.
(4) The surgery support robot according to any one of (1) to (3), wherein
   a holder is provided at a position outside a movement range of the tool driver.
(5) The surgery support robot according to any one of (1) to (4), further comprising:
   a moving mechanism that moves the turret to move the holder to the movement range of the tool driver.
(6) The surgery support robot according to (5), wherein
   the moving mechanism rotates the turret.
(7) The surgery support robot according to (5) or (6), wherein
   the turret includes a holding unit having a fan shape, and
   the moving mechanism rotates the turret about a center position of an arc of the holding unit.
(8) The surgery support robot according to (5) or (6), wherein
   the turret includes a holding unit having a circular shape, and
   the moving mechanism rotates the turret about a center position of a circular shape of the holding unit.
(9) The surgery support robot according to (5), wherein
   the moving mechanism linearly moves the turret in a direction away from the follower arm and in a direction approaching the tool driver.
(10) The surgery support robot according to any one of (5) to (9), wherein
   the turret is able to be exploded into a detachable portion and a holding unit, and
   the detachable portion is attachable to and detachable from the moving mechanism.
(11) The surgery support robot according to any one of (1) to (10), wherein
   in the turret, a first plate portion and a second plate portion are overlapped each other,
   a plurality of grooves into which the tool is inserted is formed on an outer edge of the first plate portion,
   the second plate portion is provided with a positioning unit that positions the tool inserted into the plurality of grooves, and
   the groove and the positioning unit cooperate to form the holder.
(12) The surgery support robot according to (11), wherein
   the positioning unit is a leaf spring that sandwiches the tool from both sides.
(13) The surgery support robot according to (11), wherein the positioning unit is a magnet.
(14) The surgery support robot according to any one of (4) to (13), comprising:
   a plurality of follower arms, wherein
   each of the plurality of follower arms includes the tool driver.
(15) The surgery support robot according to claim (14), wherein
   the moving mechanism rotates the turret and moves the holder to the movement range of the tool driver included in the plurality of follower arms.
(16) The surgery support robot according to (7), wherein
   the holder is formed along an arc-shaped outer edge of the holding unit having a fan shape.
(17) The surgery support robot according to (8), wherein
   the holder is formed along an outer edge of the holding unit having a circular shape.

### Reference Signs List

- 1: OPERATION DEVICE
- 5: MANIPULATION DEVICE
- 11: BASE
- 11a: TOP SURFACE
- 12: SUPPORT
- 13: BASE ARM
- 14: FOLLOWER ARM BASE
- 14a: FIRST PORTION
- 14b: SECOND PORTION
- 15: FOLLOWER ARM
- 16: CAMERA
- 17: TURRET
- 18: WHEEL
- 19: FIRST LINK
- 20: SECOND LINK
- 21, 22, 23: ROTATION AXIS
- 25: ROOT SIDE DRIVER
- 26: DISTAL END SIDE DRIVER
- 27: TOOL DRIVER
- 27a: ATTACHMENT PORTION
- 28, 29: DRIVE AXIS
- 30: TOOL
- 31: MOVING MECHANISM
- 31a: FIRST MOVING MECHANISM
- 31b: SECOND MOVING MECHANISM
- 32: DETACHABLE PORTION
- 32a: FIRST PORTION
- 32b: SECOND PORTION
- 33: HOLDING UNIT
- 33a: OUTER EDGE
- 34: DRIVE AXIS
- 35: OUTPUT SHAFT
- 36: HOLDER
- 37: FIRST PLATE PORTION
- 37a: OUTER EDGE
- 38: SECOND PLATE PORTION
- 39: GROOVE
- 40: BASE PORTION
- 40a: GROOVE
- 41: LEAF SPRING
- 42: ELASTIC PORTION
- 43: WIDENED PORTION
- 44: ROOT
- 44a: PARALLEL SURFACE
- 44b: PROTRUSION
- 45: END EFFECTOR
- 46: OPERATIONAL FIELD
- 47: MOVEMENT RANGE
- 48: DRIVE AXIS
- 49: OUTPUT SHAFT
- 50: LINEAR MOTION UNIT
- 51: IMAGE DISPLAY DEVICE
- 52: MANIPULATION UNIT
- 55: DRAPE
- 100: SURGERY SUPPORT ROBOT

## Claims

1. A surgery support robot comprising:
a base placed on a placement surface;
a base arm including a plurality of links attached to the base and connected to each other;
a follower arm base attached to the base arm;
a follower arm attached to the follower arm base and including a tool driver to and from which a tool is attachable and detachable; and
a turret on which a plurality of holders capable of holding the tool is formed and attached to the base arm or the follower arm base.

2. The surgery support robot according to claim 1, comprising
a plurality of follower arms, wherein
each of the plurality of follower arms includes the tool driver.

3. The surgery support robot according to claim 2, wherein
the plurality of the tool drivers is capable of mounting the tool held by a common turret.

4. The surgery support robot according to claim 1, wherein
a holder is provided at a position outside a movement range of the tool driver.

5. The surgery support robot according to claim 4, further comprising:
a moving mechanism that moves the turret to move the holder to the movement range of the tool driver.

6. The surgery support robot according to claim 5, wherein
the moving mechanism rotates the turret.

7. The surgery support robot according to claim 6, wherein
the turret includes a holding unit having a fan shape, and
the moving mechanism rotates the turret about a center position of an arc of the holding unit.

8. The surgery support robot according to claim 6, wherein
the turret includes a holding unit having a circular shape, and
the moving mechanism rotates the turret about a center position of a circular shape of the holding unit.

9. The surgery support robot according to claim 5, wherein
the moving mechanism linearly moves the turret in a direction away from the follower arm and in a direction approaching the tool driver.

10. The surgery support robot according to claim 5, wherein
the turret is able to be exploded into a detachable portion and a holding unit, and
the detachable portion is attachable to and detachable from the moving mechanism.

11. The surgery support robot according to claim 1, wherein
in the turret, a first plate portion and a second plate portion are overlapped each other,
a plurality of grooves into which the tool is inserted is formed on an outer edge of the first plate portion,
the second plate portion is provided with a positioning unit that positions the tool inserted into the plurality of grooves, and
the groove and the positioning unit cooperate to form the holder.

12. The surgery support robot according to claim 11, wherein
the positioning unit is a leaf spring that sandwiches the tool from both sides.

13. The surgery support robot according to claim 11, wherein
the positioning unit is a magnet.

14. The surgery support robot according to claim 5, comprising:
a plurality of follower arms, wherein
each of the plurality of follower arms includes the tool driver.

15. The surgery support robot according to claim 14, wherein
the moving mechanism rotates the turret and moves the holder to the movement range of the tool driver included in the plurality of follower arms.

16. The surgery support robot according to claim 7, wherein
the holder is formed along an arc-shaped outer edge of the holding unit having a fan shape.

17. The surgery support robot according to claim 8, wherein
the holder is formed along an outer edge of the holding unit having a circular shape.
